# EUROPÄISCHE PATENTANMELDUNG

(11) **EP 2 792 370 A1**
(43) Veröffentlichungstag der Anmeldung: **22.10.2014**
(21) Anmeldenummer: 14001417.6
(22) Anmeldetag: 17.04.2014
(51) Int. Cl.: A61K 49/00, A61K 9/00

(54) **Isotonische Lösung zum Einbringen in den Kapselsack des Auges bei ophtalmologischen Operationen**

(30) Priorität: 17.04.2013 DE 102013006597
(71) Anmelder: Corlay, Jean-Pierre, 29000 Quimper (FR)
(72) Erfinder: Corlay, Jean-Pierre, 29000 Quimper (FR)
(74) Vertreter: Czybulka, Uwe

(57) **Zusammenfassung**

Die Erfindung betrifft eine isotonische Lösung zum Einbringen in den Kapselsack des Auges bei ophthalmologischen Operationen, z. B. dem Ersatz der natürlichen Augenlinse durch eine künstliche Intraokularlinse, zum Aufrechterhalten des Augendrucks und zum Schutz der Innenseite der Cornea während der Operation, und ist dadurch gekennzeichnet, dass der isotonischen Lösung von Anfang an ein Färbemittel zugesetzt ist. Bevorzugt werden die Lösung und das Färbemittel durch Zentrifugieren homogen vermischt.

## Beschreibung

Die Erfindung bezieht sich auf eine isotonische, insbesondere gelartige viscoelastische Lösung, die bei ophthalmologischen Operationen in den Kapselsack des Auges eingebracht wird, z. B. bei dem Ersatz der natürlichen Augenlinse durch eine künstliche Intraokularlinse. Die isotonische Lösung wird in den Kapselsack eingeführt, um im Wesentlichen den inneren Augendruck aufrecht zu erhalten.

Während oder nach der Operation wird noch ein Farbstoff, z. B. Trypanblau, injiziert, der die vordere Kapsel des Auges und fibröses Bindegewebe färbt und dem Chirurgen die Kapsel klarer darstellt.

Nach der Operation wird der Kapselsack mit einer neutralen Spüllösung so lange gespült, bis kein Farbstoff in der ausgespülten Lösung sichtbar vorhanden ist. Die Operation kann dann beendet werden.

Bei dieser herkömmlichen Methode sind zwei Schritte notwendig: einmal die Injektion und gegebenenfalls Aufrechterhaltung der Strömung einer viskoelastischen isotonen Spüllösung und die erneute Injektion eines Färbemittels.

Der Erfindung liegt die Aufgabe zugrunde, dieses Verfahren zu vereinfachen.

Diese Aufgabe wird gemäss der Erfindung dadurch gelöst, dass der isotonischen Lösung von Anfang an das Färbemittel zugesetzt wird.

Das Färbemittel und die viscoelastische isotonische Lösung sind an für sich nicht miteinander mischbar. Versuche haben jedoch gezeigt, dass das Färbemittel und die viscoelastische isotonische Lösung z. B. durch Zentrifugieren homogen vermischt werden können, und die Mischung dauerhaft ist.

Das Zentrifugieren der in einem Röhrchen übereinander gestapelten viscoelastischen Lösung und des Färbemittels erfolgt in einer Zentrifuge z. B. bei Umdrehungen zwischen 1000 und 5000, bevorzugt zwischen 1500 und 3500 Umdrehungen pro Minute für eine Zeit von einigen Minuten, bis die Mischung homogen ist.

Mit der Erfindung vereinfacht sich das Verfahren bei einer ophthalmologischen Operation: Es ist durch das Injizieren der Kombinationslösung gemäss der Erfindung am Anfang der Operation nur ein Schritt notwendig, um den Augeninnendruck aufrecht zu erhalten, dem Chirurgen während der gesamten Operation ein gutes Bild des Operationsfeldes zu geben und um festzustellen, wann der Spülvorgang nach der Operation beendet werden kann.

Die isotonische Lösung gemäss der Erfindung kann steril in einem Behälter aufgenommen sein.

Es ist möglich, die Kombinationslösung in einer Spritze oder dergleichen aufzunehmen, die entsprechend steril verpackt ist und einmalig bei der Operation verwendet wird.

Zur Verbesserung der isotonischen Eigenschaften der Kombinationslösung kann dieser noch Hyaluronsäure bzw. deren Salz Natriumhyaluronat zugesetzt werden.

## Patentansprüche

1. Isotonische Lösung zum Einbringen in den Kapselsack des Auges bei ophthalmologischen Operationen, z. B. dem Ersatz der natürlichen Augenlinse durch eine künstliche Intraokularlinse, zum Aufrechterhalten des Augendrucks und zum Schutz der Innenseite der Cornea während der Operation, **dadurch gekennzeichnet, dass** der isotonischen Lösung von Anfang an ein Färbemittel zugesetzt ist.

2. Isotonische Lösung nach Anspruch 1, **dadurch gekennzeichnet, dass** die isotonische Lösung mit dem Färbemittel steril in einer Spritze aufgenommen ist.

3. Isotonische Lösung nach Anspruch 1 oder 2, **dadurch gekennzeichnet, dass** das Färbemittel Trypanblau ist.

4. Isotonische Lösung nach einem der vorhergehenden Ansprüche, **dadurch gekennzeichnet, dass** die isotonische Lösung Hyaluronsäure bzw. das Natriumsalz der Hyaluronsäure, Natriumhyaluronat, und das Färbemittel enthält.

5. Spritze zum Injizieren einer sterilen isotonischen Lösung in den Kapselsack des Auges zum Aufrechterhalten des Augendrucks und zum Schutz der Innenseite der Cornea bei ophtalmologischen Operationen, **dadurch gekennzeichnet, dass** die Spritze die isotonische Lösung und ein Färbemittel in einer homogenen Mischung enthält und die Spritze steril verpackt ist.

6. Verfahren zum Herstellen einer isotonischen Lösung nach Anspruch 1, **dadurch gekennzeichnet, dass** die isotonische Lösung und das Färbemittel durch Zentrifugieren homogen vermischt werden.

7. Verfahren nach Anspruch 6, **dadurch gekennzeichnet, dass** das Färbemittel und die Lösung bei Umdrehungen zwischen 1000 und 5000, bevorzugt bei 1500 und 3500 Umdrehungen homogen vermischt werden.2
